# EUROPEAN PATENT APPLICATION

(11) **EP 4 119 035 A1**
(43) Date of publication of application: **18.01.2023**
(21) Application number: 22184458.2
(22) Date of filing: 12.07.2022
(51) Int. Cl.: A61B 3/12

(54) **MULTISPECTRAL FUNDUS IMAGING**

(30) Priority: 12.07.2021 US 202163220803 P; 27.06.2022 US 202217809048
(71) Applicant: Welch Allyn, INC., Skaneateles Falls, NY 13153-0220 (US)
(72) Inventor: HUNTER, Vivian L, Skaneateles Falls, 13153-0220 (US); GUO, Lei, Skaneateles Falls, 13153-0220 (US); HART, Allen R, Skaneateles Falls, 13153-0220 (US); LANE, John A, Skaneateles Falls, 13153-0220 (US)
(74) Representative: Spencer, James Michael

(57) **Abstract**

A fundus imager (100) includes a handheld housing that supports a lighting unit (112) configured to illuminate an eye fundus. The lighting unit (112) includes one or more light-emitting diodes (220). The housing further supports a camera (104) configured to capture one or more images of the eye fundus, and a display (108) configured to display the one or more images of the eye fundus. The fundus imager (100) captures at least one multispectral fundus image using the camera (104), and displays the at least one multispectral fundus image on the display (108).

## Description

A fundus imager is a device that captures images of the fundus and other structures of the eye. The fundus images can be used to determine the health of the retina, and to screen for diseases such as macular degeneration, diabetic retinopathy, glaucoma, and papilledema.

Drusen are yellow deposits under the retina that are made up of lipids and proteins. The presence of drusen are considered a sign of macular degeneration. Drusen may sometimes go undetected in color fundus images. Moreover, diseases such as macular degeneration may go undetected due to low compliance of through eye exams and poor access to eye care.

In general terms, the present disclosure relates to a technique within a fundus imager to screen for eye diseases closer to a single point of care. In one possible configuration, the fundus imager generates multispectral images that allow for extraction of details such as drusen and other artifacts that are not detectable in color fundus images. Various aspects are described in this disclosure, which include, but are not limited to, the following aspects.

One aspect relates to a fundus imager comprising: a handheld housing supporting: a lighting unit configured to illuminate an eye fundus, the lighting unit including one or more light-emitting diodes; a camera configured to capture one or more images of the eye fundus; and a display configured to display the one or more images of the eye fundus; at least one processing device; and at least one computer readable data storage device storing software instructions that, when executed by the at least one processing device, cause the fundus imager to: capture at least one multispectral fundus image using the camera; and display the at least one multispectral fundus image on the display.

Another aspect relates to a method of eye disease screening, comprising: flooding an eye fundus with illumination in one or more predetermined spectral bands; capturing at least one multispectral fundus image; analyzing the at least one multispectral fundus image for identification of one or more artifacts symptomatic of an eye disease; and providing an output based on the identification of one or more artifacts in the at least one multispectral fundus image, wherein the output includes at least one of a diagnosis of the eye disease and a recommendation to follow up with an eye care professional.

Another aspect relates to a method of eye disease screening, comprising: flooding an eye fundus with illumination; filtering light reflected from the eye fundus to capture a multispectral fundus image; analyzing the multispectral fundus image for identification of one or more artifacts symptomatic of an eye disease; displaying the multispectral fundus image on a display supported by a housing that is sized and shaped to be handheld and portable; and providing on the display an output based on the identification of one or more artifacts in the multispectral fundus image, wherein the output includes at least one of a diagnosis of the eye disease and a recommendation to follow up with an eye care professional.

Examples will now be further described with reference to the accompanying drawings, in which:
FIG. 1 schematically illustrates an example of a fundus imager that is operable by a clinician for capturing and viewing a fundus image of a patient.
FIG. 2 is a top isometric view of an example of the fundus imager of FIG. 1 from the perspective of a clinician.
FIG. 3 is a bottom isometric view of the fundus imager of FIG. 1 from the perspective of a clinician.
FIG. 4 is another top isometric view of the fundus imager of FIG. 1 from the perspective of a patient.
FIG. 5 is another bottom isometric view of the fundus imager of FIG. 1 from the perspective of a patient.
FIG. 6 is a left side view of the fundus imager of FIG. 1.
FIG. 7 is a right side view of the fundus imager of FIG. 1.
FIG. 8 is a bottom view of the fundus imager of FIG. 1.
FIG. 9 is a top view of the fundus imager of FIG. 1.
FIG. 10 is a front view of the fundus imager of FIG. 1.
FIG. 11 is a rear view of the fundus imager of FIG. 1.
FIG. 12 is a side view showing the fundus imager of FIG. 1 positioned against the patient's head.
FIG. 13 is another isometric view of an example of the fundus imager of FIG. 1 with a portion of a housing of the funds imager removed.
FIG. 14 is an isometric view of an example of a camera in the fundus imager of FIG. 1, and an enlarged view of a lighting unit.
FIG. 15 is an isometric view of another example of a lighting unit for capturing multispectral fundus images in the fundus imager of FIG. 1.
FIG. 16 schematically illustrates an example of a spectral filter for capturing multispectral fundus images in the fundus imager of FIG. 1.
FIG. 17 illustrates an example of a method of eye disease screening performed by the fundus imager of FIG. 1.
FIG. 18 shows a comparison of a color fundus image with multispectral fundus images of an eye exhibiting drusen-induced retinal pigment epithelium (RPE) degeneration.
FIG. 19 shows another comparison of a color fundus image with multispectral fundus images of an eye exhibiting subretinal hemorrhage.
FIG. 20 shows another comparison of a color fundus image with multispectral fundus images of an eye exhibiting epiretinal membrane.
FIG. 21 schematically illustrates training a deep learning algorithm used by the fundus imager of FIG. 1 to predict age related macular degeneration.
FIG. 22 schematically illustrates an example of using the deep learning algorithm of FIG. 19 to predict severity likelihoods for age related macular degeneration.
FIG. 23 illustrates another example of a method of eye disease screening performed by the fundus imager of FIG. 1.
FIG. 24 schematically illustrates an example of a computing device of the fundus imager of FIG. 1.

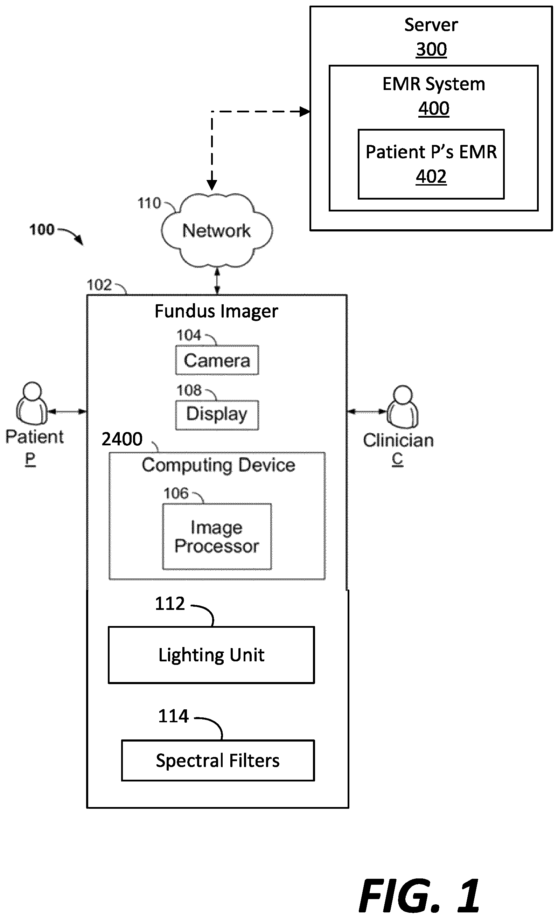

FIG. 1 schematically illustrates an example of a fundus imager 100. As shown in FIG. 1, the fundus imager 100 is operable by a clinician C to capture and view fundus images of a patient P. As used herein, "fundus" refers to the eye fundus, which includes the retina, optic nerve, macula, vitreous, choroid, and posterior pole. In certain aspects, the fundus imager 100 includes components similar to those described in U.S. Patent Application No. 16/443,234, filed on June 17, 2019, which is hereby incorporated by reference in its entirety.

The fundus imager 100 can be used by the clinician C to screen, diagnose, and/or monitor the progression of one or more eye diseases, such as macular degeneration, diabetic retinopathy, hypertension, glaucoma, papilledema, and the like. Additionally, the fundus imager 100 can also be used to capture and display images of the eye for other purposes. For example, the fundus imager 100 can also be used to capture images of the anterior portion of the eye including structures in front of the vitreous humor such as the cornea, iris, ciliary body, and lens.

In some examples, the clinician C is an eye care professional such as an optometrist or ophthalmologist who uses the fundus imager 100 to screen, diagnose, and/or monitor the progression of one or more eye diseases. In further examples, the clinician C can be a medical professional who is not trained as an eye care professional such as a general practitioner or primary care physician. In such examples, the fundus imager 100 can be used to screen for one or more eye diseases in a general practice medical office or other type of medical clinic. In further examples, the clinician C can be a non-medical practitioner such as an optician who can help fit eyeglasses, contact lenses, and other vision-correcting devices such that the fundus imager 100 can be used to screen for one or more eye diseases in a retail clinic. In yet further examples, the fundus imager 100 can be used by the patient P to take fundus images of their eyes without assistance of the clinician C such that the fundus imager 100 can be used as a home device to screen, diagnose, and/or monitor for eye diseases such as macular degeneration.

In examples where the clinician C is not an eye care professional, the fundus imager 100 can be configured to screen for eye diseases in a general practice medical office, retail clinic, or patient home by capturing one or more fundus images, detecting the presence of one or more artifacts in the fundus images, and providing a recommended diagnosis for an eye disease or a recommendation to follow up with an eye care professional. The fundus imager 100 can include software algorithms that can analyze the captured fundus images for detection of artifacts such as drusen. Thus, the fundus imager 100 can help users who are not trained eye care professionals to screen for one or more eye diseases, such as, for example, age related macular degeneration.

One technique for fundus imaging requires mydriasis, or the dilation of the patient's pupil, which can be painful and/or inconvenient to the patient P. The fundus imager 100 does not require a mydriatic drug to be administered to the patient P before imaging, although the fundus imager 100 can image the fundus if a mydriatic drug has been administered.

The fundus imager 100 includes a computing device 2400 having at least an image processor 106. The fundus imager 100 further includes a camera 104 in communication with the computing device 2400, and a display 108 in communication with the computing device 2400. The camera 104 captures digital images of the eye fundus of the patient P, and the display 108 displays the captured digital images for viewing by the clinician C.

The camera 104 is in communication with the image processor 106. The camera 104 is a digital camera that includes a lens, an aperture, and a sensor array. The lens can be a variable focus lens, such as a lens moved by a step motor, or a fluid lens, also known as a liquid lens. The camera 104 is configured to capture images of the fundus one eye at a time. In other examples, the camera 104 is configured to capture an image of both eyes substantially simultaneously. In such examples, the fundus imager 100 can include two separate cameras, one for each eye.

The display 108 is in communication with the image processor 106. In the examples shown in the figures, the display 108 is supported by a housing 102. In other examples, the display 108 can connect to an image processor that is external of the fundus imager 100, such as a separate smart phone, tablet computer, or external monitor. The display 108 functions to display the images produced by the camera 104 in a size and format readable by the clinician C. In some examples, the display 108 is a liquid crystal display (LCD) or active matrix organic light emitting diode (AMOLED) display. In some examples, the display 108 is touch sensitive.

The fundus imager 100 further includes a lighting unit 112 that illuminates the eye fundus of the patient P within specific wavelength ranges across the electromagnetic spectrum allowing the camera 104 to capture multispectral fundus images. As will be described in more detail, the lighting unit 112 includes one or more light-emitting diodes (LEDs) that are configured to flash or shine light within one or more spectral bands, and thereby flood the eye fundus with illumination allowing the camera 104 to produce multispectral fundus images.

The fundus imager 100 can further include one or more spectral filters 114. As will be described in more detail below, the spectral filters can be used by the fundus imager 100 to filter the light reflected from the eye fundus of the patient P to produce multispectral fundus images.

In some examples, the lighting unit 112 can be used with or without the one or more spectral filters 114. Similarly, in some further examples, the one or more spectral filters 114 can be used with or without the lighting unit 112.

As used herein, multispectral imaging refers to a technique for capturing fundus images within specific wavelength ranges across the electromagnetic spectrum. Thus, multispectral imaging captures fundus images in wavelength bands that are narrower than broadband white light sources such as a halogen bulb or white LED. The multispectral images can be captured by using the lighting unit 112 to illuminate the eye fundus under particular wavelength ranges of light such as in the blue, green, and red spectral bands, and also in wavelength ranges beyond the visible light ranges such as near infrared and infrared wavelength ranges. The separate narrower wavelength images (e.g., in the blue, green, red, near-infrared spectral bands) are displayed by the fundus imager 100 to more clearly show disease states compared to color fundus images captured using full white light conventional light sources.

The multispectral fundus imaging allows for extraction of details such as drusen or retinoblastomas that may not be visible in color fundus images. For example, the multispectral fundus images can help intensify the contrast between healthy tissue and lesions. Thus, the multispectral fundus images produced by the fundus imager 100 can more easily be analyzed by the clinician him/herself, and also by computer algorithms for eye disease screening.

As shown in FIG. 1, the fundus imager 100 is connected to a network 110. The fundus imager 100 can upload the fundus images captured by the camera 104, including the multispectral fundus images, to a remote server 300 via the connection to the network 110. In at least some examples, the remote server 300 is a cloud server or similar type of server.

In some examples, the remote server 300 includes an electronic medical record (EMR) system 400 (alternatively termed electronic health record (EHR)). Advantageously, the remote server 300 can automatically store the fundus images of the patient P in an electronic medical record 402 or electronic health record of the patient P located in the EMR system 400.

In examples where the clinician C is not an eye care professional, such as when the fundus imager 100 is used for screening for eye diseases in general practice medical offices, in retail clinics, or in the patient P's home, the fundus images stored in the electronic medical record 402 of the patient P can be accessed by an overread clinician who is an eye care professional. Thus, the fundus images can be accessed and viewed on another device by a remotely located clinician. Accordingly, in some examples, the clinician who operates the fundus imager 100 is different from the clinician who evaluates the fundus images.

The network 110 may include any type of wireless network, wired network, or any combination of wireless and wired networks. Wireless connections can include cellular network connections, including connections made using protocols such as 802.11a, b, and/or g. In some examples, a wireless connection can be accomplished directly between the fundus imager 100 and an external display device using one or more wired or wireless protocols, such as Bluetooth, Wi-Fi, radio-frequency identification (RFID), or Zigbee. Other configurations are possible.

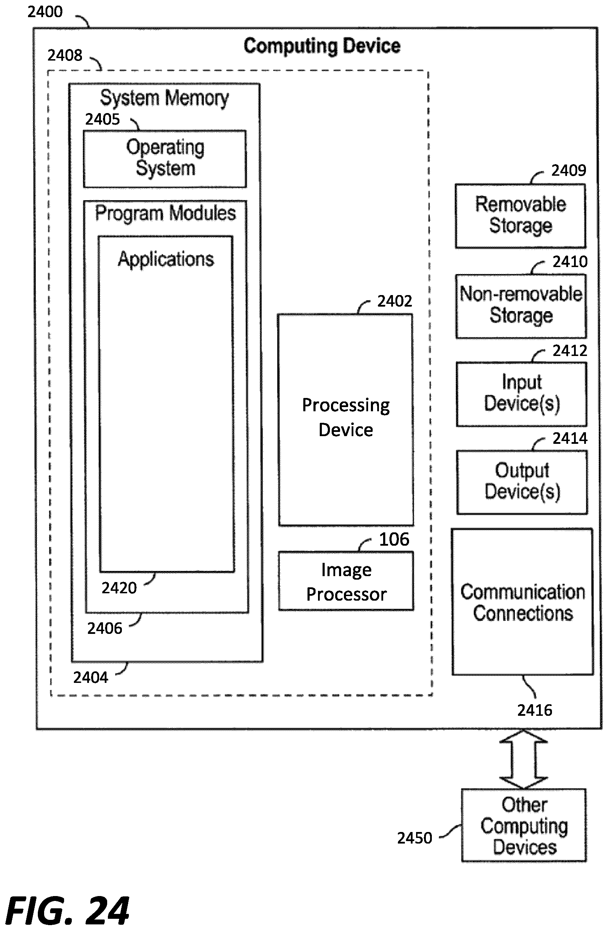

The image processor 106 is coupled to the camera 104 and configured to communicate with the network 110 and display 108. The image processor 106 can regulate the operation of the camera 104. Components of an example of the computing device 2400 are shown in more detail in FIG. 24, which will be described further below.

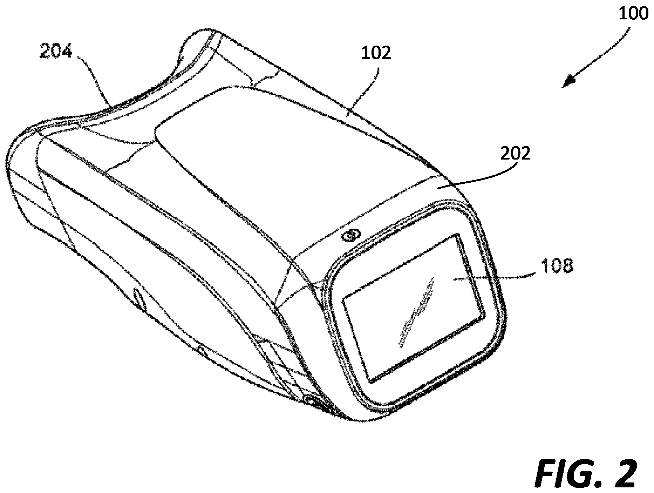

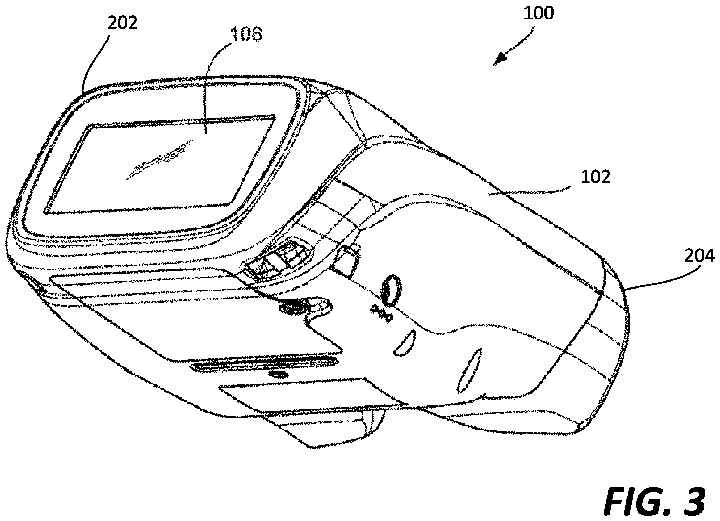

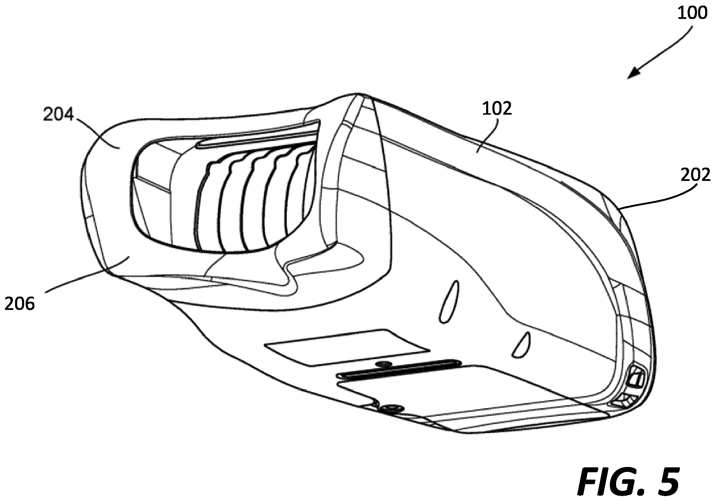

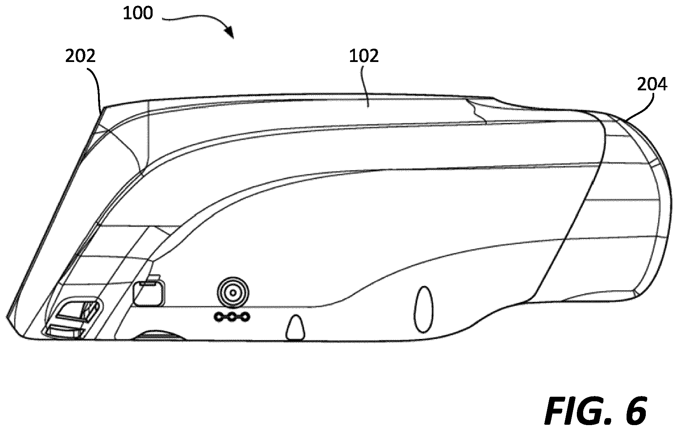

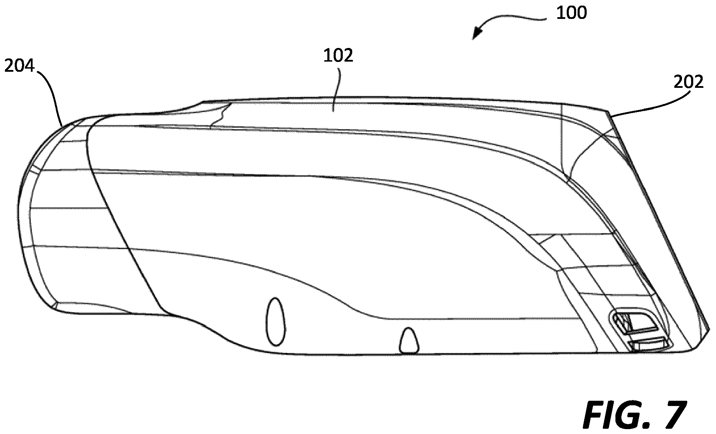

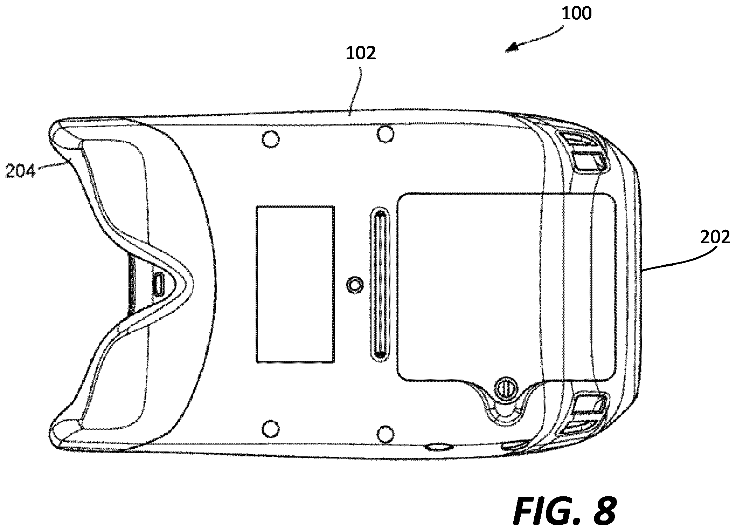

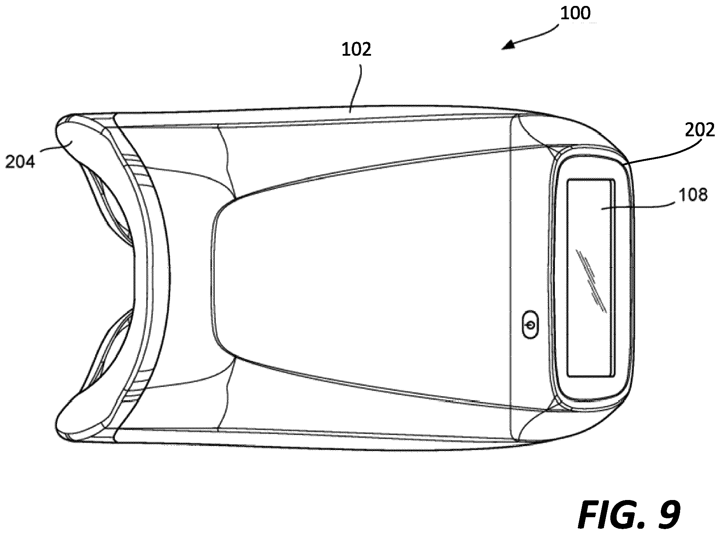

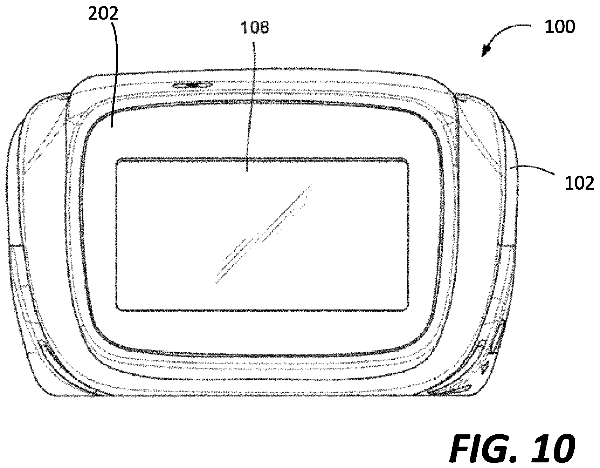

As shown in FIG. 1, the fundus imager 100 includes a housing 102 that supports the computing device 2400, camera 104, display 108, lighting unit 112, and spectral filters 114. An example of the housing 102 is shown in FIGS. 2-12.

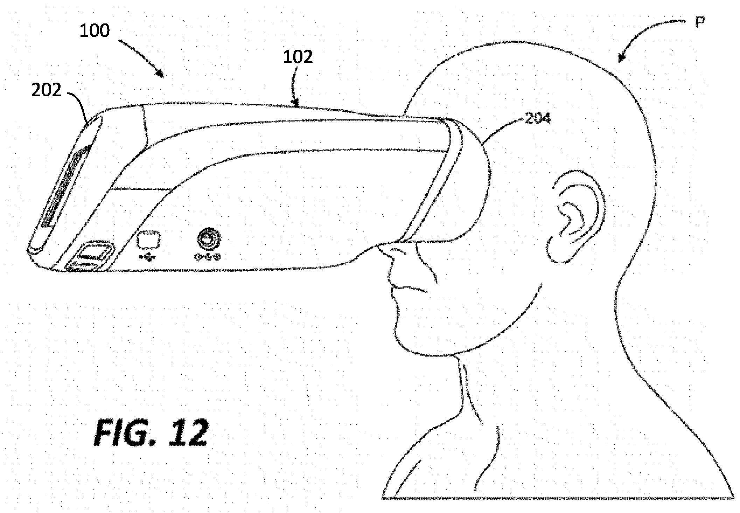

Referring now to FIGS. 2-12, the housing 102 supports the display 108 at a first end 202. The housing 102 further includes an opposite, second end 204 configured to engage the face of the patient P (see FIG. 12). The housing 102 is sized and shaped to be handheld and portable.

The display 108 is configured to display the captured fundus images of the left and right eyes of the patient P. Additionally, the display 108 may also be configured to display controls for capturing the fundus images in examples where the display 108 is a touchscreen. The housing 102 can additionally support one or more user input buttons near display 108. The display 108 can be used to initiate the image capture sequence, as described herein. Thus, the fundus imager 100 is configured such that the clinician C can implement one or more automatic and/or manual workflows for the capture of fundus images of the patient P's eyes.

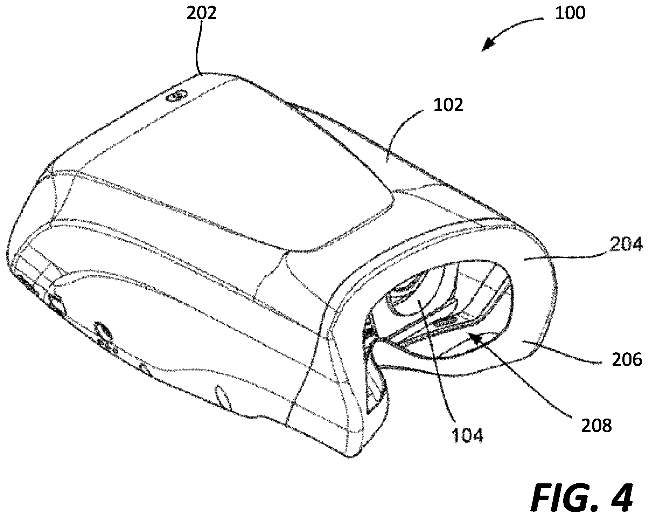

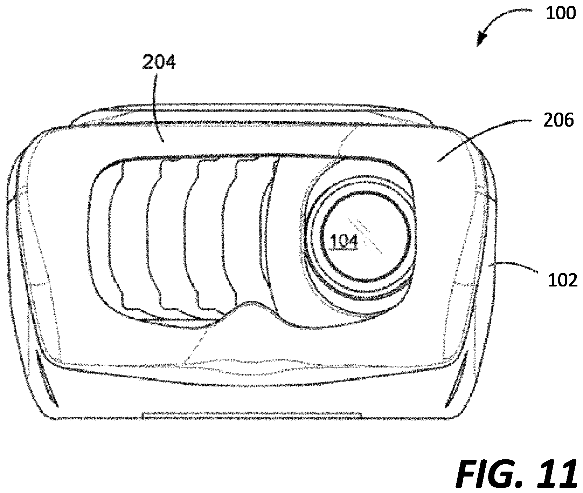

As shown in FIGS. 4 and 11, the second end 204 of the housing 102 includes a surface 206 for engaging the patient P's head. For example, the surface 206 can be positioned again the patient P's face and to surround both eyes of the patient P, as shown in FIG. 12.

The housing 102 further defines a cavity 208 at the second end 204. The camera 104 is partially positioned within the cavity 208, and is configured to be moved in at least three directions to accomplish fundus imaging of both the left and right eyes of the patient P while the housing 102 of the fundus imager 100 is positioned and held against the patient P's face.

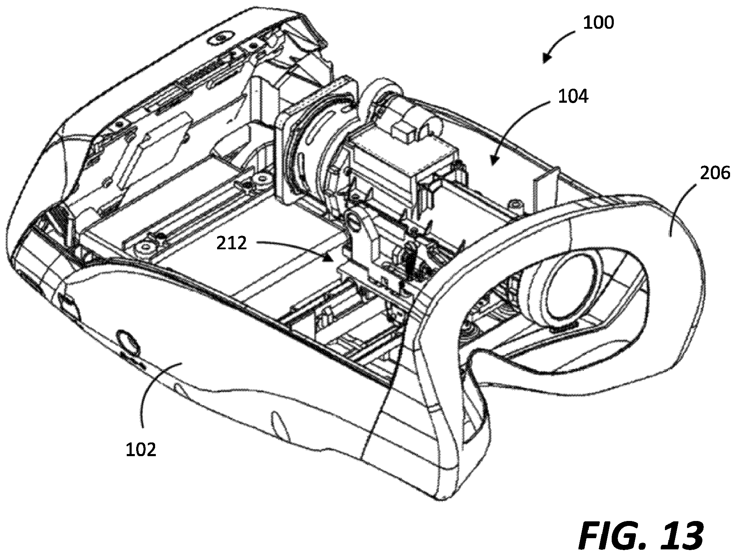

FIG. 13 is another isometric view of an example of the fundus imager 100 with a portion of the housing 102 removed. FIG. 13 illustrates at least some of the internal components of the fundus imager 100. As shown in FIG. 13, the fundus imager 100 includes the camera 104 that is moved along multiple axes by a base assembly 212. The internal components of the fundus imager 100 may be similar to those described in U.S. Patent Number 10,993,613, issued on May 4, 2021, which is hereby incorporated by reference in its entirety.

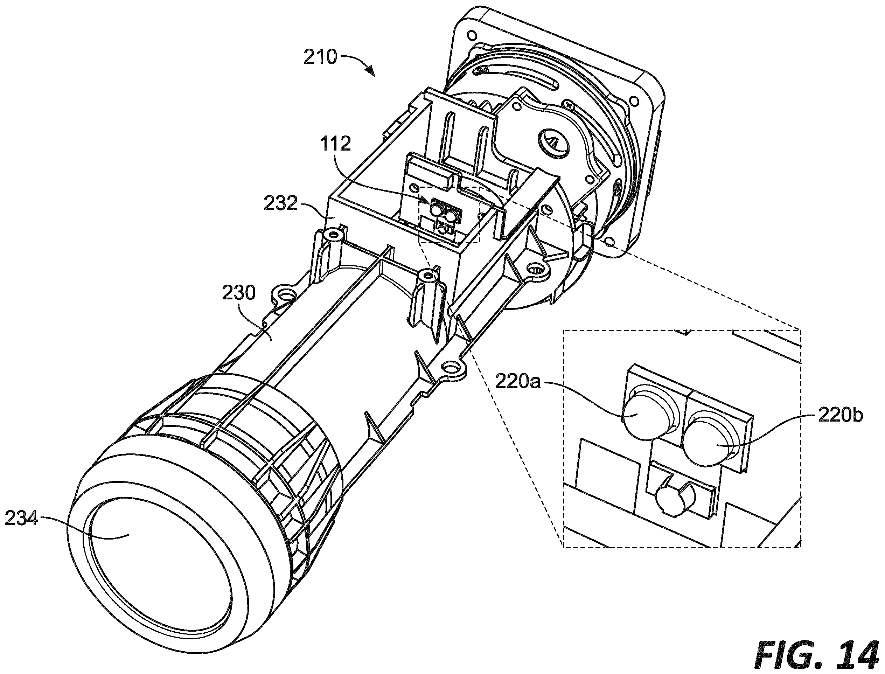

FIG. 14 is an isometric view of an example of the camera 104, and an enlarged view of the lighting unit 112. In this example, the camera 104 includes an optical lens barrel 230 that extends from an electrical housing 232. The optical lens barrel 230 includes a lens 234 positioned at a distal end of the optical lens barrel 230. The lighting unit 112 is positioned inside the electrical housing 232, and is configured to illuminate the eye fundus of the patient P.

The lighting unit 112 includes one or more light-emitting diodes (LEDs) 220. The LEDs 220 can be configured to emit light within specific wavelengths across the electromagnetic spectrum to allow the fundus imager 100 to perform a multispectral imaging technique.

In the example shown in FIG. 14, the lighting unit 112 includes two LEDs 220a, 220b that are positioned side by side. As an illustrative example, the LED 220a can be a visible light LED that is configured to illuminate light in one or more of the visible light spectrums, and the LED 220b can be an infrared or near-infrared LED that is configured to illuminate light in the infrared or near-infrared light spectrums. In alternative examples, the lighting unit 112 can include more than two LEDs, or can include fewer than two LEDs (i.e., a single LED).

In some examples, the lighting unit 112 can include a single, multicolor LED. The multicolor LED can have multiple channels each capable of independent and tandem operation. As an example, the lighting unit 112 can have a three-channel RGB LED that emits light in the red, green, and blue visible light spectrums. Alternative arrangements are possible.

In the example shown in FIG. 14, the LEDs 220a, 220b are positioned on a surface that is aligned with the optical lens barrel 230 to illuminate light through the lens 234 such that the light from the LEDs 220a, 220b is directly aimed toward the patient P's eyes. Alternatively, the LEDs 220a, 220b can be positioned elsewhere inside the fundus imager 100 such that the light from the LEDs 220a, 220b is indirectly aimed at the patient P's eyes.

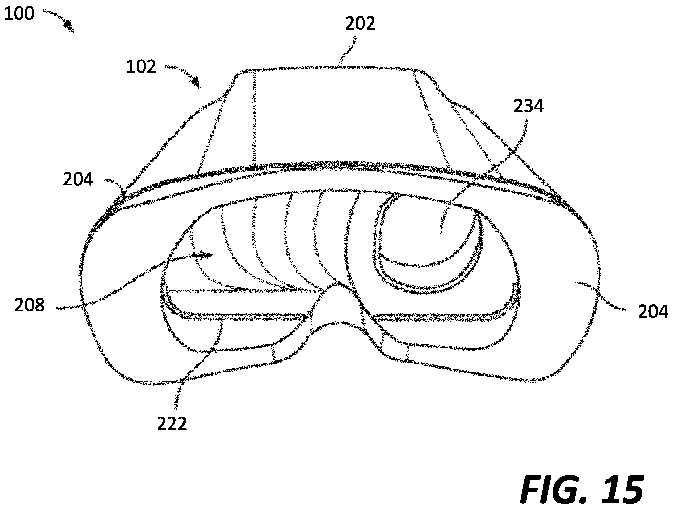

FIG. 15 is an isometric view of another example of the lighting unit 112 for the fundus imager 100. In this example, the lighting unit 112 includes an illumination ring 222 orientated along an interior surface of the cavity 208. The illumination ring 222 is generated by one or more LEDs, and is configured to flood the cavity 208 with light, and thereby indirectly illuminate the eye fundus of the patient P within one or more spectral bands. The illumination ring 222 can surround, or at least partially surround, the periphery of the optical lens barrel 230.

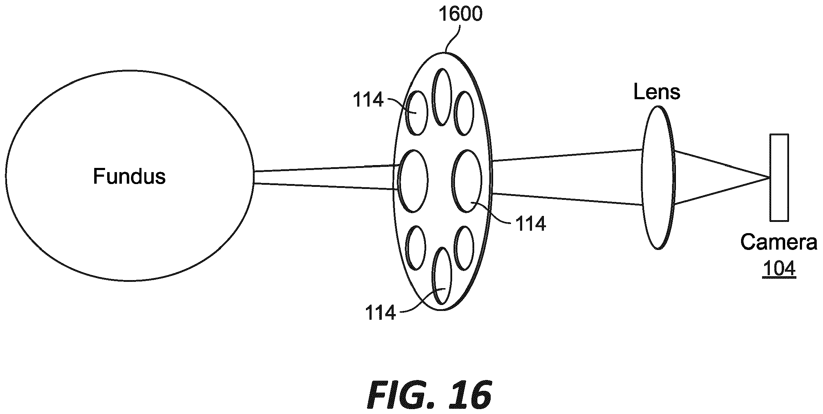

FIG. 16 schematically illustrates an example of the spectral filters 114 that can be used for capturing multispectral fundus images in the fundus imager 100. As described above, reflected white light may be separated by the spectral filters 114 to produce the multispectral fundus images. In some examples, the spectral filters 114 are tunable filters.

In the example of FIG. 16, the spectral filters 114 are positioned on a filter wheel 1600, which is positioned into the optical path of the camera 104. The filter wheel 1600 works synchronically with the camera 104 such that each exposure of the camera 104 corresponds to a particular type of spectral filter 114, which yields a multispectral fundus image.

In other examples, the spectral filters 114 can include electronically-tunable filters. Examples of electronically-tunable filters include liquid crystal tunable filters (LCTF), and acousto-optical tunable filters (AOTF). Additionally, in some examples, beam splitters that include dichroic filters can be used to generate multispectral fundus images. In some further examples, a plurality of spectral bands (e.g., red, green, blue, etc.) are captured all in one image frame, and are then separated in post processing performed by the image processor 106.

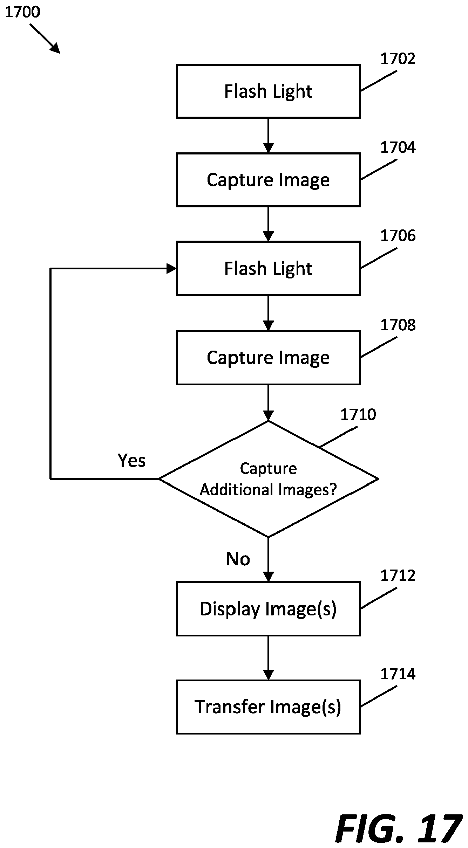

FIG. 17 illustrates an example of a method 1700 of eye disease screening that can be performed by certain examples of the fundus imager 100. As described above, the fundus imager 100 is a handheld device that the clinician C can position against the patient P's face to capture one or more eye fundus images. The method 1700 is performed by the fundus imager 100 in response to receiving a signal to initiate an image capture workflow. The signal is generated by the clinician C selecting a control on the display 108 or housing 102 when the fundus imager 100 is appropriately positioned against the face of the patient P, such as shown in FIG. 12.

The method 1700 includes an operation 1702 of flashing a first type of light, and an operation 1704 of capturing a first image. Operations 1702 and 1704 are performed substantially simultaneously such that the first image is captured while the first type of light is being flashed. In operation 1702, the first type of light is a white light and the first image captured in operation 1704 is a color fundus image. The first type of light can be captured using a standard illumination light source of the fundus imager 100. In alternative examples, the first type of light can be captured using the lighting unit 112 of the fundus imager 100.

The method 1700 next includes an operation 1706 of flashing a second type of light, and an operation 1708 of capturing a second image. Operations 1706 and 1708 are performed substantially simultaneously such that the second image is captured while the second type of light is being flashed. In operation 1706, the second type of light is within a predetermined wavelength range, and the image captured in operation 1708 is a multispectral fundus image. The second type of light can be captured using the lighting unit 112 of the fundus imager 100.

In some examples, the second type of light is in the blue spectral band (e.g., having a wavelength between approximately 450 - 500 nm). In some examples, the second type of light is in the green spectral band (e.g., having a wavelength between approximately 500 - 570 nm). In some examples, the second type of light is in the yellow spectral band (e.g., having a wavelength between approximately 570 - 590 nm). In some examples, the second type of light is in the orange spectral band (e.g., having a wavelength between approximately 590 - 610 nm). In some examples, the second type of light is in the red spectral band (e.g., having a wavelength between approximately 610 - 700 nm). In some examples, the second type of light is in the near-infrared spectral band (e.g., having a wavelength between approximately 700-1400 nm).

The second type of light is flashed using the lighting unit 112 in accordance with the examples described above. For example, one or more of the LEDs 220 shown in FIG. 14 can be controlled by the computing device 2400 during operation 1706 to emit the second type of light within a predetermined wavelength range of the electromagnetic spectrum. Alternatively, the illumination ring 222 shown in FIG. 15 can be controlled by the computing device 2400 during operation 1706 to emit the second type of light within the predetermined wavelength range.

As an illustrative example, the LEDs 220 or illumination ring 222 can be controlled to emit light in the blue spectral band (e.g., wavelength between approximately 450 - 500 nm), in the green spectral band (e.g., wavelength between approximately 500 - 570 nm), in the red spectral band (e.g., wavelength between approximately 610 - 700 nm), in the near-infrared spectral band (e.g., wavelength between approximately 700-1400 nm), and so on.

In some examples, operation 1706 includes flashing light from a combination of two or more of the LEDs. As an illustrative example, operation 1706 can include flashing light from the LED 220a in the blue spectral band (e.g., wavelength between approximately 450 - 500 nm), while simultaneously flashing light from the LED 220b in the red spectral band (e.g., wavelength between approximately 610 - 700 nm) or in the near-infrared spectral band (e.g., wavelength between approximately 700-1400 nm). Additional combinations are possible such that different combinations of colored LEDS can be combined in a single flash.

Still referring to FIG. 17, the method 1700 includes an operation 1710 of determining whether additional fundus images are needed according to the image capture workflow selected by the clinician C. In some examples, the image capture workflow may require one color fundus image captured according to operations 1702, 1704, and only one multispectral image captured according to operations 1706, 1708. Accordingly, when no additional multispectral images are needed (i.e., "No" at operation 1710), the method 1700 proceeds to operation 1712.

In some further examples, the image capture workflow may require a sequence of multispectral fundus images that are each captured in different predetermined spectral bands. Accordingly, when additional multispectral images are needed (i.e., "Yes" at operation 1710), the method 1700 repeats the operations 1706, 1708 to capture multiple multispectral fundus images using different colored LEDS or combinations of colored LEDs.

As an illustrative example, a sequence of multispectral fundus images can be captured by capturing a first multispectral fundus image in the blue spectral band (e.g., wavelength between approximately 450 - 500 nm), capturing a second multispectral fundus image in the green spectral band (e.g., wavelength between approximately 500 - 570 nm), capturing a third multispectral fundus image in the red spectral band (e.g., wavelength between approximately 610 - 700 nm), capturing a fourth multispectral fundus image in the near-infrared spectral band (e.g., wavelength between approximately 700-1400 nm), and so on. Additionally, the sequence of multispectral fundus images can includes images that are captured by combining two or more different spectral bands (e.g., flashing light in the blue spectral band while simultaneously flashing light in the red spectral band) to capture a multispectral fundus image.

Certain spectral bands are optimal for detecting certain disease states because each spectral band penetrates retinal tissue at a different depth. Shorter wavelengths reflect from a top layer of the retinal tissue, while longer wavelengths penetrate further into the retinal tissue before scattering back. Thus, shorter wavelengths such as those in the blue and green spectral bands can be used to image artifacts on the top layer of the retinal tissue, while longer wavelengths such as those in the red or near infra-red spectral bands can be used to image artifacts that are present in deeper layers of the retinal tissue and at the back surface of the retina. Accordingly, the image capture workflow can select one or more spectral bands for capturing one or more multispectral fundus images that optimally identify fluid build up and drusen which are symptomatic of age related macular degeneration, and to determine whether blood vessels are bleeding.

Table 1 provides an illustrative example of spectral bands of light, layer of retinal tissue penetration, and artifacts and disease states that can be detected therefrom.

**Table 1.**

| **Spectral Band** | **Layer of Detection** | **Artifact / Disease State** |
|---|---|---|
| Blue light (486nm) | Inner retina | • Epiretinal membrane |
| | | • Macular pigment changes |
| | | • rNFL thinning |
| | | • Subretinal drusenoid deposits (SDD) |
| Green light (518nm) | Middle retina layers | • Drusen (inner layers) |
| | | • Blood Vessels (RVO, CRVO & BRVO) |
| Near-infrared (820nm) | Outer retina | • Drusen (outer layers) |
| | | • Choroidal Vessels |
| | | • Retinal Pigment Epithelium (RPE) |
| | | • Geographic Atrophy |
| | | • Macular Edema |

In some examples, each fundus image captured in operations 1704, 1708 is tagged to identify the type of light source that was used to capture the image. For example, the color fundus images can be tagged to identify that the standard illumination light source of the fundus imager 100 was used to capture them. Similarly, the multispectral fundus images can be tagged to identify which of the LEDs 220a, 220b, or combinations thereof, were used.

Still referring to FIG. 17, when no additional multispectral images are needed (i.e., "No" at operation 1710), the method 1700 proceeds to operation 1712 of displaying the captured fundus images on the display 108. In some examples, operation 1712 includes displaying the captured fundus images on the display 108 individually. In alternative examples, operation 1712 includes displaying the captured fundus images on the display 108 side by side.

For example, a color fundus image captured in operation 1704 can be displayed next to a multispectral fundus image captured in operation 1708. In this example, the color fundus image can act as a reference image for the clinician C who may be more familiar with reviewing color fundus images, and the multispectral fundus image can help the clinician C identify artifacts such as drusen or retinoblastomas that may not be easily recognizable in the color fundus image, and visualize a contrast between healthy retina tissue and lesions. In at least some examples, the multispectral fundus image(s) when displayed in the display 108 of the fundus imager 100 can help the clinician C identify symptoms of age related macular degeneration.

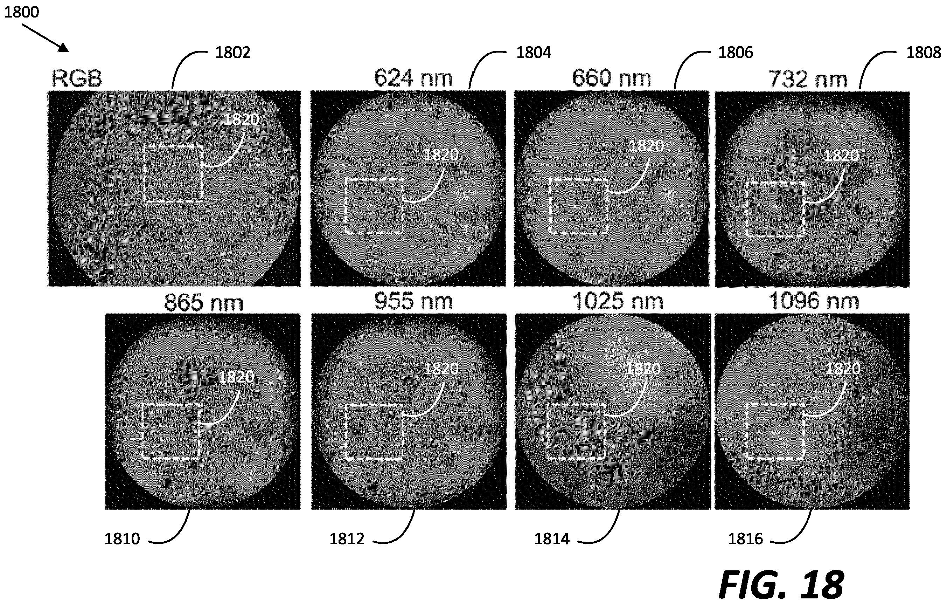

FIG. 18 shows a comparison 1800 of a color fundus image 1802 with multispectral fundus images 1804-1816 of an eye exhibiting drusen-induced retinal pigment epithelium (RPE) degeneration. The color fundus image 1802 and multispectral fundus images 1804-1816 each include a boundary 1820 that helps to identify the subretinal hemorrhage in the fundus images. As shown in FIG. 18, the drusen-induced RPE degeneration is difficult to see in the color fundus image 1802, while it is more clearly noticeable in the multispectral fundus images corresponding to mid and long wavelengths in which light penetrates deeper into the retinal tissue, such as in the multispectral fundus images 1808, 1810.

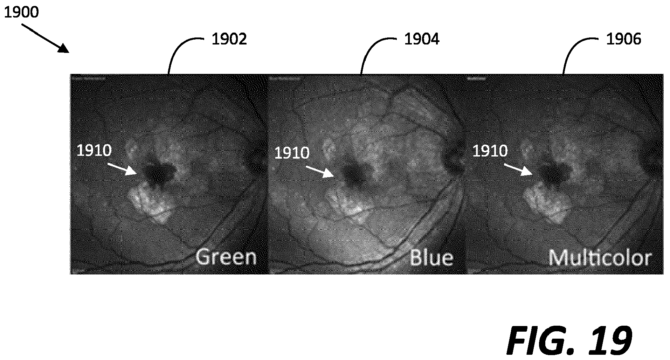

FIG. 19 shows another comparison 1900 of a color fundus image 1906 with multispectral fundus images 1902, 1904 of an eye exhibiting subretinal hemorrhage. In this example, the multispectral fundus image 1902 is captured in the green spectral band, while the multispectral fundus image 1904 is captured in the blue spectral band. The color fundus image 1906 and multispectral fundus images 1902, 1904 each include a marker 1910 that helps to identify the subretinal hemorrhage in the fundus images. As shown in the example provided in FIG. 19, the subretinal hemorrhage is more visible in the multispectral fundus image 1902 than in the multispectral fundus image 1904 and the color fundus image 1906.

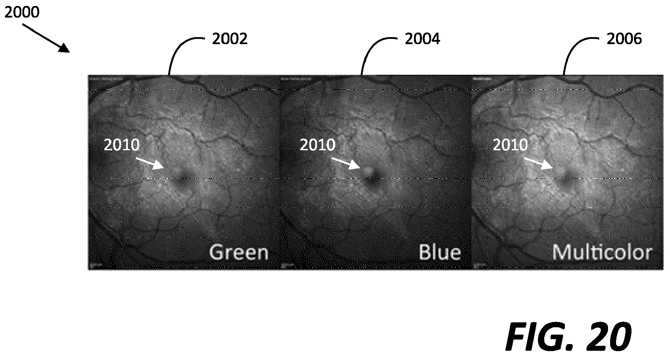

FIG. 20 shows another comparison 2000 of a color fundus image 2006 with multispectral fundus images 2002, 2004 of an eye exhibiting epiretinal membrane. In this example, the multispectral fundus image 2002 is captured in the green spectral band, while the multispectral fundus image 2004 is captured in the blue spectral band. The color fundus image 2006 and multispectral fundus images 2002, 2004 each include a marker 2010 that helps to identify the epiretinal membrane in the fundus images. As shown in the example provided in FIG. 20, the epiretinal membrane is more visible in the multispectral fundus image 2004 than in the multispectral fundus image 2002 and the color fundus image 2006.

In some examples, one or more computer implemented algorithms can select an optimal multispectral fundus image from a sequence of multispectral fundus images by analyzing each multispectral fundus image, and determining which one shows the best or highest level of contrast for a particular type of artifact or eye condition. The computer implemented algorithms can utilize deep learning or artificial intelligence assistance. Thus, in some examples, operation 1712 can include displaying only the optimal multispectral fundus image on the display 108, or displaying the optimal multispectral fundus image next to a color fundus image for comparison.

In some examples, method 1700 can further include performing one or more computer implemented algorithms on the fundus imager 100 that analyze the multispectral fundus images to identify artifacts and adverse eye conditions in the images. The one or more computer implemented algorithms can utilize deep learning and artificial intelligence assistance. As described above, the multispectral fundus images can help the one or more computer implemented algorithms more efficiently and accurately analyze the fundus images because the multispectral fundus images can provide sharper contrasts for objects in the images.

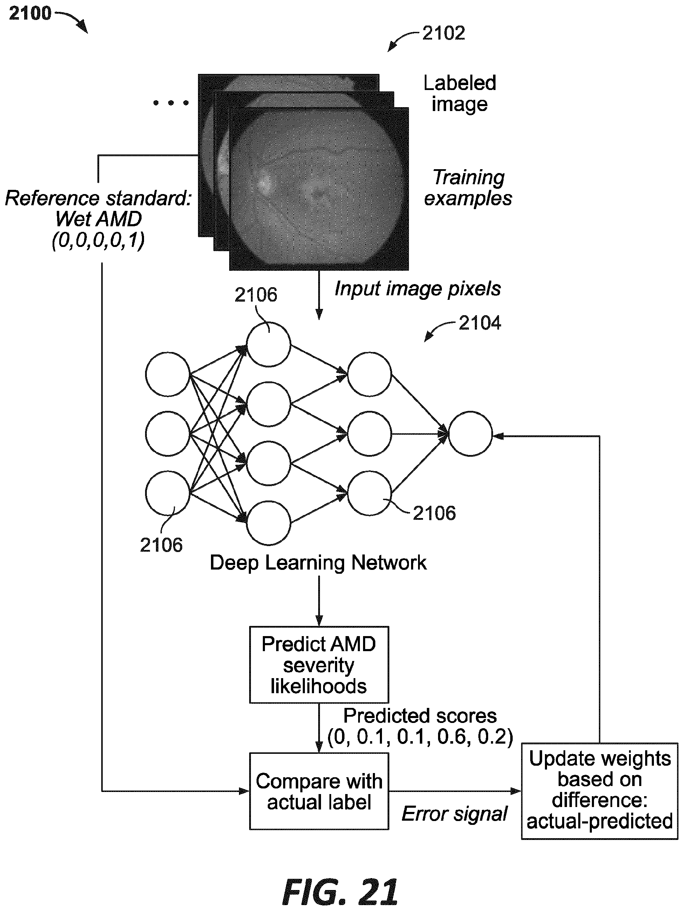

FIG. 21 schematically illustrates an example of training a deep learning algorithm 2100 that can be used by the fundus imager 100 to predict age related macular degeneration severity likelihoods. As shown in FIG. 21, a plurality of training examples 2102 are input into a deep learning network 2104. The training examples 2102 are labeled fundus images that depict various degrees of severity of age related macular degeneration, or that depict no age related macular degeneration. The training examples 2102 are labeled as such.

The deep learning network 2104 includes a plurality of neural network nodes 2106 each having a weight factor that in combination can be used to predict age related macular degeneration likelihoods from the training examples. The predictions are compared to the actual labels on the fundus images. If differences exist between the predictions and actual labels, the weight factors of the neural network nodes 2106 are updated by the deep learning algorithm.

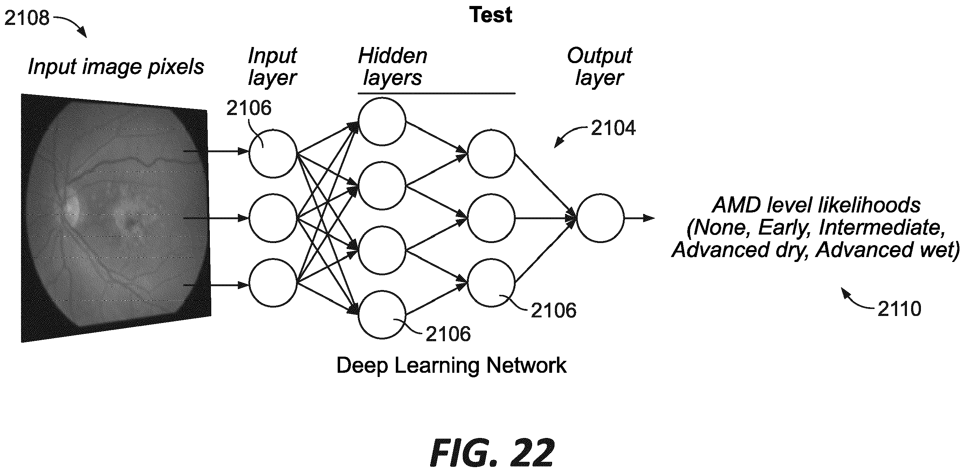

FIG. 22 illustrates an example of using the deep learning algorithm 2100 to predict severity likelihoods for age related macular degeneration after the algorithm has been trained. As shown in FIG. 22, input images 2108 are entered into the deep learning network 2104. The input images 2108 can include one or more color fundus images and multispectral fundus images taken from a given eye of the patient P using the fundus imager 100. The deep learning network 2104 analyzes the layers of the input images 2108 using the plurality of neural network nodes 2106 to predict a likelihood level 2110 for age related macular degeneration such as none, early stage, intermediate stage, advanced dry stage, advanced wet stage, and the like.

The fundus imager 100 can display one or more markers over the artifacts and adverse conditions in one or more fundus images displayed on the display 108. In some examples, the markers can be displayed over the color fundus image. In other examples, the markers can be displayed over the multispectral fundus image. The markers can be especially helpful for clinicians who are not eye care professionals, and accordingly may improve screening for eye diseases in general practice medical offices, retail clinics, and the like.

Additionally, the fundus imager 100 can display one or more boundaries or heatmaps that identify areas of the fundus image for further review and analysis (see, for example, the boundaries 1820 shown in FIG. 18). Also, the fundus imager can analyze the fundus images for calculating an Early Treatment Diabetic Retinopathy Study (ETDRS) score.

Still referring to FIG. 17, the method 1700 includes an operation 1714 of transferring the color fundus image and at least one multispectral fundus image to another device. Operation 1714 can include uploading the fundus images to the remote server 300 via the connection to the network 110. The remote server 300 can store the fundus images in the electronic medical record 402 of the patient P located in the EMR system 400. An overread clinician, such as an optometrist or ophthalmologist, can thereafter access the fundus images to provide a professional diagnosis. In some examples, one or more computer implemented algorithms are executed by the remote server 300 to provide an automated diagnosis based on the fundus images.

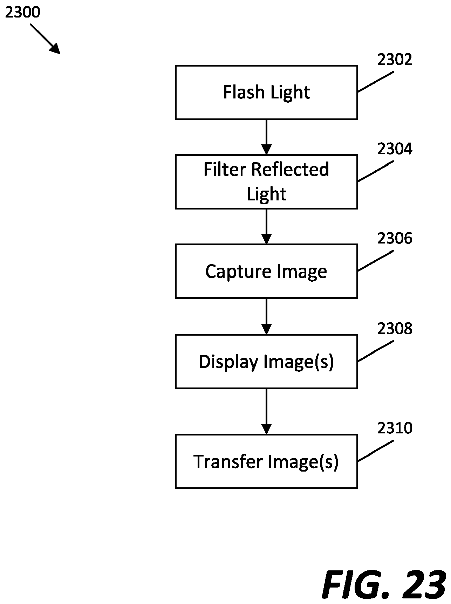

FIG. 23 illustrates another example of a method 2300 of eye disease screening that can be performed by certain examples of the fundus imager 100. In the method 2300, a multispectral image is produced and displayed on the display 108 of the fundus imager 100 by filtering light from a standard illumination light source of the fundus imager 100.

As shown in FIG. 23, the method 2300 includes an operation 2302 of flashing light. In some examples, the light is flashed in operation 2302 using the standard illumination light source of the fundus imager 100. In other examples, the light can be flashed as a white light using the lighting unit 112 in accordance with the examples described above.

Next, the method 2300 includes an operation 2304 of filtering the light reflected back from the eye fundus using the spectral filters 114. As described above, the spectral filters 114 are positioned in the optical path of the camera 104, and can include a filter wheel 1600 (shown in FIG. 16), electronically-tunable filters such as liquid crystal tunable filters (LCTF) and acousto-optical tunable filters (AOTF), and beam splitters that include dichroic filters.

In some examples, one or more spectral filters 114 can be combined to provide custom filtering. For example, a custom filtering can allow the blue and red spectral bands of reflected light to pass through the spectral filters 114, while blocking the green spectral band.

Next, the method 2300 includes an operation 2306 of capturing a multispectral fundus image, and thereafter an operation 2308 of displaying the multispectral fundus image on the display 108 of the fundus imager 100. Like in the method 1700 described above, the method 2300 may be repeated to capture a plurality of multispectral fundus images. In some examples, each fundus image captured in operation 2306 is tagged to identify the type of filter that was used to capture the image. In operation 2308, the captured fundus images can be displayed on the display 108 individually, or the captured fundus images can be displayed on the display 108 side by side such that a color fundus image can be displayed next to a multispectral fundus image.

The method 2300 further can further include an operation 2310 of transferring the one or more multispectral fundus images to another device. Operation 2310 can be substantially similar to operation 1714, which is described above with reference to FIG. 17.

In alternative examples, a method of producing a multispectral fundus image can include capturing a plurality of spectral bands (e.g., red, green, blue, etc.) all in one image frame, and then separating the spectral bands in post processing performed by the image processor 106.

FIG. 24 schematically illustrates an example of the computing device 2400 of the fundus imager 100, with which the various methods, functions, and features of the present disclosure may be practiced. The computing device 2400 includes the image processor 106, and at least one processing device 2402 that can be used, either separately or in combination with the image processor 106, to regulate the operation of the camera 104, display 108, lighting unit 112, spectral filters 114, and other components of the fundus imager 100.

The computing device 2400 further includes a system memory 2404. The system memory 2404 may comprise, but is not limited to, volatile storage (e.g., random access memory), non-volatile storage (e.g., read-only memory), flash memory, or any combination of such memories. The system memory 2404 may include an operating system 2405 and one or more program modules 2406 suitable for running software applications 2420. The operating system 2405, for example, may be suitable for controlling the operation of the fundus imager 100.

Furthermore, embodiments of the disclosure may be practiced in conjunction with a graphics library, other operating systems, or any other application program and is not limited to any particular application or system. This basic configuration is illustrated in FIG. 24 by those components within a dashed line 2408. The computing device 2400 may have additional features or functionality. For example, the computing device 2400 can include additional data storage devices, including a removable storage device 2409 and a non-removable storage device 2410.

Embodiments of the present disclosure may be practiced in an electrical circuit comprising discrete electronic elements, packaged or integrated electronic chips containing logic gates, a circuit utilizing a microprocessor, or on a single chip containing electronic elements or microprocessors. For example, embodiments of the present disclosure may be practiced via a system-on-a-chip (SOC) where each or many of the components illustrated in FIG. 24 may be integrated onto a single integrated circuit. When operating via an SOC, the functionality, described herein, may be operated via application-specific logic integrated with other components of the computing device 2400 on the single integrated circuit.

The computing device 2400 can be connected to one or more input device(s) 2412, such as the display 108 when configured as a touchscreen. Also, the computing device 2400 can be connected to one or more output device(s) 2414 such as the display 108, speakers, and the like. The computing device 2400 can be connected to additional input/output devices.

The computing device 2400 may include one or more communication connections 2416 allowing communications with other computing devices 2450, and with the network 110. Examples of communication connections 2416 include RF transmitter, receiver, and/or transceiver circuitry; universal serial bus (USB), parallel, and/or serial ports.

The term computer readable storage device as used herein may include non-transitory computer storage media. Computer storage media may include volatile and nonvolatile, removable and non-removable media implemented in any method or technology for storage of information, such as computer readable instructions, data structures, or program modules. The system memory 2404, the removable storage device 2409, and the non-removable storage device 2410 are all examples of computer readable storage devices. Computer readable storage devices may further include RAM, ROM, electrically erasable read-only memory (EEPROM), flash memory or other memory technology, or any article of manufacture which can be used to store information and which can be accessed by the computing device 2400. Additionally, any such computer readable storage devices may be considered part of the computing device 2400.

Communication media may be embodied by computer readable instructions, data structures, program modules, or other data in a modulated data signal, such as a carrier wave or other transport mechanism, and includes any information delivery media. The term "modulated data signal" may describe a signal that has one or more characteristics set or changed in such a manner as to encode information in the signal. By way of example, communication media may include wired media such as a wired network or direct-wired connection, and wireless media such as acoustic, radio frequency (RF), infrared, and other wireless media.

Embodiments of the present invention may be utilized in various distributed computing environments where tasks are performed by remote processing devices, such as one or more devices linked through the network 110 in a distributed computing environment.

The block diagrams depicted herein are just examples. There may be many variations to these diagrams described therein without departing from the spirit of the disclosure. For instance, components may be added, deleted or modified.

The systems and method described herein result in a significant technical advantage. For example, the fundus imager 100 is programmed to more efficiently detect and/or identify eye diseases using the acquired multispectral fundus images. This allows the fundus imager 100 to more efficiently analyze fundus images for eye disease screening.

Embodiments of the disclosure can be described with reference to the following numbered clauses, with preferred features laid out in the dependent clauses:
1. A fundus imager, comprising:
   a handheld housing supporting:
      a lighting unit configured to illuminate an eye fundus, the lighting unit including one or more light-emitting diodes;
      a camera configured to capture one or more images of the eye fundus; and
      a display configured to display the one or more images of the eye fundus;
   at least one processing device; and
   at least one computer readable data storage device storing software instructions that, when executed by the at least one processing device, cause the fundus imager to:
      capture at least one multispectral fundus image using the camera; and
      display the at least one multispectral fundus image on the display.
2. The fundus imager of clause 1, wherein the software instructions, when executed by the at least one processing device, further cause the fundus imager to:
   capture a sequence of multispectral fundus images, each multispectral fundus image being captured within a different spectral band.
3. The fundus imager of clause 2, wherein the sequence of multispectral fundus images ranges from a blue spectral band to a near-infrared spectral band.
4. The fundus imager of clause 2, wherein the software instructions, when executed by the at least one processing device, further cause the fundus imager to:
   select an optimal multispectral fundus image from the sequence of multispectral fundus images by determining which multispectral fundus image from the sequence of multispectral fundus images shows a highest level of contrast for one or more artifacts.
5. The fundus imager of clause 4, wherein the one or more artifacts are drusen.
6. The fundus imager of clause 1, wherein the software instructions, when executed by the at least one processing device, further cause the fundus imager to:
   capture at least one color fundus image; and
   display the at least one color fundus image on the display.
7. The fundus imager of clause 6, wherein the at least one color fundus image is displayed next to the at least one multispectral fundus image.
8. The fundus imager of clause 1, wherein the software instructions, when executed by the at least one processing device, further cause the fundus imager to:
   analyze the at least one multispectral fundus image for identification of artifacts symptomatic of an eye disease; and
   provide an output based on an identification of one or more artifacts in the at least one multispectral fundus image, wherein the output includes at least one of a diagnosis of the eye disease and a recommendation to follow up with an eye care professional.
9. The fundus imager of clause 8, wherein the software instructions, when executed by the at least one processing device, further cause the fundus imager to:
   display a marker over an artifact identified in the at least one multispectral fundus image.
10. The fundus imager of clause 1, wherein the software instructions, when executed by the at least one processing device, further cause the fundus imager to:
   upload the at least one multispectral fundus image to a remote server for storage in an electronic medical record that is accessible by an overread clinician.
11. The fundus imager of clause 1, wherein the lighting unit is configured to flash light within one or more predetermined spectral bands to produce the at least one multispectral fundus image.
12. The fundus imager of clause 11, wherein the lighting unit is positioned inside a cavity of defined by the housing, and wherein the lighting unit is configured to flood the eye fundus with illumination in the one or more predetermined spectral bands.
13. The fundus imager of clause 1, further comprising:
   one or more spectral filters configured to filter light reflected from the eye fundus to produce the at least one multispectral fundus image.
14. The fundus imager of clause 13, wherein the one or more spectral filters include at least one of a liquid crystal tunable filter (LCTF), an acousto-optical tunable filter (AOTF), and beam splitters positioned in an optical path of the camera.
15. The fundus imager of clause 1, wherein a plurality of spectral bands are captured in a single image frame, and the plurality of spectral bands are then separated in post processing to capture the at least one multispectral fundus image.
16. A method of eye disease screening, comprising:
   flooding an eye fundus with illumination in one or more predetermined spectral bands;
   capturing at least one multispectral fundus image;
   analyzing the at least one multispectral fundus image for identification of one or more artifacts symptomatic of an eye disease; and
   providing an output based on the identification of one or more artifacts in the at least one multispectral fundus image, wherein the output includes at least one of a diagnosis of the eye disease and a recommendation to follow up with an eye care professional.
17. The method of clause 16, further comprising:
   capturing a color fundus image; and
   displaying the at least one multispectral fundus image next to the color fundus image.
18. The method of clause 16, further comprising:
   displaying at least one of a marker over an artifact in the at least one multispectral fundus image and a boundary identifying an area in the at least one multispectral fundus for review.
19. A method of eye disease screening, comprising:
   flooding an eye fundus with illumination;
   filtering light reflected from the eye fundus to capture a multispectral fundus image;
   analyzing the multispectral fundus image for identification of one or more artifacts symptomatic of an eye disease;
   displaying the multispectral fundus image on a display supported by a housing that is sized and shaped to be handheld and portable; and
   providing on the display an output based on the identification of one or more artifacts in the multispectral fundus image, wherein the output includes at least one of a diagnosis of the eye disease and a recommendation to follow up with an eye care professional.
20. The method of clause 19, further comprising:
   capturing a color fundus image; and
   displaying the multispectral fundus image next to the color fundus image.

## Claims

1. A fundus imager, comprising:
a handheld housing supporting:
a lighting unit configured to illuminate an eye fundus, the lighting unit including one or more light-emitting diodes;
a camera configured to capture one or more images of the eye fundus; and
a display configured to display the one or more images of the eye fundus;
at least one processing device; and
at least one computer readable data storage device storing software instructions that, when executed by the at least one processing device, cause the fundus imager to:
capture at least one multispectral fundus image using the camera; and
display the at least one multispectral fundus image on the display.

2. The fundus imager of claim 1, wherein the software instructions, when executed by the at least one processing device, further cause the fundus imager to:
capture a sequence of multispectral fundus images, each multispectral fundus image being captured within a different spectral band.

3. The fundus imager of claim 2, wherein the sequence of multispectral fundus images ranges from a blue spectral band to a near-infrared spectral band.

4. The fundus imager of claim 2, wherein the software instructions, when executed by the at least one processing device, further cause the fundus imager to:
select an optimal multispectral fundus image from the sequence of multispectral fundus images by determining which multispectral fundus image from the sequence of multispectral fundus images shows a highest level of contrast for one or more artifacts.

5. The fundus imager of claim 4, wherein the one or more artifacts are drusen.

6. The fundus imager of claim 1, wherein the software instructions, when executed by the at least one processing device, further cause the fundus imager to:
capture at least one color fundus image; and
display the at least one color fundus image on the display.

7. The fundus imager of claim 6, wherein the at least one color fundus image is displayed next to the at least one multispectral fundus image.

8. The fundus imager of claim 1, wherein the software instructions, when executed by the at least one processing device, further cause the fundus imager to:
analyze the at least one multispectral fundus image for identification of artifacts symptomatic of an eye disease; and
provide an output based on an identification of one or more artifacts in the at least one multispectral fundus image, wherein the output includes at least one of a diagnosis of the eye disease and a recommendation to follow up with an eye care professional.

9. The fundus imager of claim 8, wherein the software instructions, when executed by the at least one processing device, further cause the fundus imager to:
display a marker over an artifact identified in the at least one multispectral fundus image.

10. The fundus imager of claim 1, wherein the software instructions, when executed by the at least one processing device, further cause the fundus imager to:
upload the at least one multispectral fundus image to a remote server for storage in an electronic medical record that is accessible by an overread clinician.

11. The fundus imager of claim 1, wherein the lighting unit is configured to flash light within one or more predetermined spectral bands to produce the at least one multispectral fundus image.

12. The fundus imager of claim 11, wherein the lighting unit is positioned inside a cavity of defined by the housing, and wherein the lighting unit is configured to flood the eye fundus with illumination in the one or more predetermined spectral bands.

13. The fundus imager of claim 1, further comprising:
one or more spectral filters configured to filter light reflected from the eye fundus to produce the at least one multispectral fundus image.

14. The fundus imager of claim 13, wherein the one or more spectral filters include at least one of a liquid crystal tunable filter (LCTF), an acousto-optical tunable filter (AOTF), and beam splitters positioned in an optical path of the camera.

15. The fundus imager of claim 1, wherein a plurality of spectral bands are captured in a single image frame, and the plurality of spectral bands are then separated in post processing to capture the at least one multispectral fundus image.
